# EUROPEAN PATENT SPECIFICATION

(11) **EP 2 794 030 B1**
(45) Date of publication and mention of the grant of the patent: **15.03.2017**
(21) Application number: 12813193.5
(22) Date of filing: 17.12.2012
(51) Int. Cl.: A61Q 17/00, A61Q 11/00, A61K 31/23, A61K 8/37, A61K 8/42, A61K 31/235, A61K 31/166

(54) **COMPOSITIONS COMPRISING GALLATES AND GALLAMIDES**
ZUSAMMENSETZUNGEN MIT GALLATEN UND GALLAMIDEN
COMPOSITIONS COMPRENANT DES GALLATES ET DES GALLAMIDES

(30) Priority: 21.12.2011 US 201113333209
(43) Date of publication of application: 29.10.2014
(73) Proprietor: Colgate-Palmolive Company, New York, NY 10022 (US)
(72) Inventor: JARACZ, Stanislav, Somerset, New Jersey 08873 (US); XU, Guofeng, Plainsboro, New Jersey 08536 (US); MILLER, Steven, Skillman, New Jersey 08558 (US); LEIGH, Leonora, Piscataway New Jersey 08854 (US); PICQUET, Guillaume, Long Valley New Jersey 07853 (US); DU-THUMM, Laurence, Princeton New Jersey 08540 (US)
(74) Representative: Jenkins, Peter David
(86) International application number: PCT/US2012/070062
(87) International publication number: WO 2013/096182

(56) References cited:
- JP-A- 2005 213 194
- US-A1- 2003 105 027
- US-A1- 2006 293 257
- US-A1- 2010 330 001
- US-A1- 2011 135 772
- US-A1- 2012 321 566

## Description

### BACKGROUND

Polyphenols are a large family of natural compounds found in a wide variety of plants, fruits and vegetables. In the recent years, polyphenols have attracted a great deal of attention in the scientific community and in the media due to their potential health benefits. For example, polyphenols are known to be strong antioxidants, and as a result are believed to have potential anti-inflammatory and anti-cancer properties. Green tea is relatively high in polyphenols, including epigallocatechin gallate (EGCG), also known as epigallocatechin 3-gallate, which is the ester of epigallocatechin and gallic acid. EGCG is known for its antioxidant properties and also for its antibacterial properties. By suppressing oral bacteria, EGCG can suppress gingivitis, and oral care products comprising EGCG are known.

Antimicrobial peptides (AMPs) such as LL-37 are produced and released by mammalian cells as a defense against bacterial infection. AMPs are drawn to bacteria by an electrostatic attraction to the anionic bacterial membrane. They are amphiphillic, which allows AMPs to traverse and disrupt membrane, creating pores and killing the bacteria. The AMPs are specific to bacteria, as human cells are stabilized by cholesterol and proteins. WO 2010/121213 posits that natural polyphenols such as EGCG, having trihydroxybenzoate moieties, in addition to their antioxidant properties, are effective as antibacterial agents by stimulating the production of certain endogenous AMPs, particularly LL-37 peptide, from epithelial cells, which can kill bacteria in the oral cavity, thus promoting healthy periodontal condition.

EGCG has some disadvantages, however. First, due to its complicated structure, it cannot be synthesized cheaply and efficiently, while extracts from natural sources may be highly variable as to quality, purity and concentration. Secondly, EGCG is unstable and degrades relatively quickly forming brown products, probably related to oxidation processes. EGCG can be stabilized using acids, e.g., ascorbic acid, but this requires a pH < 4, which is unacceptable for a dentifrice. At pH=7, ascorbic acid is ineffective in protecting EGCG. In dentifrice, the onset of discoloration can be delayed by addition of stannous chloride and decreasing water content, but this has a negative impact on efficacy.

A great many synthetic compounds having gallate moieties have been disclosed, e.g., in Kazi, et al., Anticancer Res. (2004) 24: 943-954 as proteasome inhibitors for potential anticancer use, in WO 2010/043631 as protein kinase inhibitors potentially useful for treating cancer, Down's syndrome or sickle cell anemia, and in US 2010/00137194 as plasminogen activator inhibitor-1 (PAI-1) inhibitors potentially useful to modulate lipid metabolism. These references do not however disclose or teach that the compounds described have potential antibacterial activity or utility for topical application or in oral or personal care products, or that they have any advantages over EGCG for such use. JP 2005-213194 discloses an antibacterial polyphenol derivative. US 2010/330001 relates to oral antibacterial compositions comprising trihydroxybenzoate derivatives. US 2003/105027 describes nutritional supplement compositions. US 2006/293257 describes topical compositions and methods for the treatment of scar tissue. US 2011/135772 discloses a skin care agent and cosmetic and/or dermopharmaceutical compostion obtained thereof.

Accordingly, there is a need for compounds having antibacterial activity similar to or better than EGCG, but which are cheaper to manufacture and are more stable in formulations.

### SUMMARY

The invention provides an oral care composition for topical use comprising a gallate or gallamide compound which is a polysubstituted cycloalkyl, wherein the substituents are selected from hydroxy, hydroxymethyl, fluoro, chloro, amino, nitro, or a moiety of formula -X-(CO)-(3,4,5-trihydroxyphenyl), wherein X is selected from O and NH, provided that the substituents comprise at least two moieties of formula -X-(CO)-(3,4,5-trihydroxyphenyl) attached to adjacent carbons, in free or in orally or topically acceptable salt form, wherein the concentration of the gallate or gallamide compound is from 0.001 to 5% (the compounds sometimes hereinafter referred to for convenience as cycloalkyl gallates or CAGs). The invention further provides a composition as defined herein for use in i) inhibiting microbial biofilm formation in the oral cavity, (ii) reducing plaque accumulation, (iii) reducing or inhibiting gingivitis, (iv) reducing or inhibiting formation of dental caries, (v), reducing, repairing or inhibiting pre-carious lesions of the enamel, (vi) cleaning the teeth and oral cavity, and/or (vii) promoting systemic health. The use typically comprises topically administering an effective amount of these gallate or gallamide compounds to a subject in need thereof. The oral care composition may be provided as a toothpaste or mouthwash. The invention additionally provides a composition for topical use (e.g. an oral care or personal care composition) comprising a gallate compound which is a polysubstituted cycloalkyl wherein the substituents are selected from hydroxy, hydroxymethyl, fluoro, chloro, amino, nitro, or a moiety of formula -X-(CO)-(3,4,5-trihydroxyphenyl), wherein X is O, provided that the substituents comprise at least two moieties of formula -X-(CO)-(3,4,5-trihydroxyphenyl) attached to adjacent carbons; in free or in topically acceptable salt form, wherein the concentration of the gallate or gallamide compound is from 0.001 to 5%.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

The identification of EGCG analogs useful for oral care products such as dentifrice or mouthwash involved first identifying the structural elements associated with activity then screening selected compounds. The design based on structure-activity-relationship studies available in literature, molecular modeling, conformational analysis, stability prediction and considerations related to ease of synthesis. Information on synthetic analogs of EGCG is rather rare and typically involves minimum alteration to EGCG structure. Similar natural products having only one 1,2,3-trihydroxybenzene ring (e.g. epigallocatechin, catechin gallate) were significantly less potent in LL-37 production than EGCG. Furthermore, three hydroxy groups on the benzene ring and their 1,2,3 relative orientation also appears essential for its activity. We performed molecular modeling with Chem3D software using molecular mechanics. Conformational analysis of EGCG generates two structures, which show that the two 1,2,3-trihydroxybenzene units are either stacked to each other (syn-conformer) or nearly 90 degrees in relative orientation (anti-conformer). This suggests that functional EGCG analog must have 2 trihydroxybenzene units close to each other.

In order to increase stability of the material against oxidation, it is desirable to add electron-withdrawing substituent on the aromatic ring. The carbonyl group lends itself as a potential connecting group to attach the two 1,2,3-trihydroxybenzene units together via a designed scaffold. Such a connection could be facilitated using ketone, amide or ester. The scaffold is designed such that it forces the two 1,2,3-trihydroxybenzene units to be close to each other providing the health benefits of natural EGCG without discoloration and stability issues. Such a scaffold is preferably a cycloalkyl or cycloheteroalkyl, with at least two hydroxy or amino groups on adjacent carbons to form esters or amide with gallic acid, and optionally further substituted with hydroxy, chlorine, fluorine, nitro, amino, and/or additional substituents bearing 1,2,3,-trihydroxy benzene moieties. So for example the scaffold could be cyclopentane, cyclohexane, trans-1,2-dihydroxycyclohexane, cis-1,2-dihydroxycyclohexane, trans-1,2-cyclohexanediamine, cis-1,2-cyclohexanediamine, cis-1,2-dihydroxycyclopentane, cis-1,2-cyclopentanediamine, hexahydro-pyridazine or pyrazolidine, or could be based on a monosaccaride ring, e.g., furanose or pyranose, which would have cost advantages for larger scale production.

Based on these considerations and *in silico* experiments, compounds are screened and further characterized as described in the examples, particularly with respect to their ability to elicit LL-37 peptide from epithelial cells and their anti-oxidative capability.

The invention therefore provides a composition for topical use, comprising a concentration of from 0.001 to 5% of a gallate or gallamide compound which is a polysubstituted cycloalkyl or heterocvcloalkyl (e.g. cyclopentyl, cyclohexyl, tetrahydrofaran, or tetrahydropyran), wherein die substituents are selected from hydroxy, hydroxymethyl, fluoro, chloro, amino, nitro, or a moiety of formula -X- (CO)-(3,4,5-trihydroxyphenyl), wherein X is selected from O, provided that the substituents comprise at least two moieties of formula - X-(CO)-(3,4,5-trihydroxyphenyl) attached to adjacent carbons; in free or in orally or topically acceptable salt form; e.g., a composition, wherein the gallate compound is a compound of Formula 1, wherein X is O, A is cyclopentyl or cyclohexyl, optionally substituted with one or more substituents selected from one or more of hydroxy, hydroxymethyl, and moiety of formula -X-(CO)-(3,4,5-trihydroxyphenyl); in free or topically acceptable salt form. The invention therefore provides furthermore an oral care composition for topical use (Composition 1 comprising a concentration of from 0.001 to 5% of a gallate or gallamide compound which is a polysubstituted cycloalkyl or heterocycloalkyl (e.g. cyclopentyl, cyclohexyl, tetrahydrofuran, or tetrahydropyran), wherein the substituents are selected from hydroxy, hydroxymethyl, fluoro, chloro, amino, nitro, or a moiety of formula -X-(CO)-(3,4,5-trihydroxyphenyl), wherein X is selected from O and NH, provided that the substituents comprise at least two moieties of formula -X-(CO)-(3,4,5-trihydroxyphenyl) attached to adjacent carbons; in free or in orally or topically acceptable salt form; i.e. an oral care composition, comprising a concentration of from 0.001 to 5% of a gallate or gallamide compound of Formula 1: 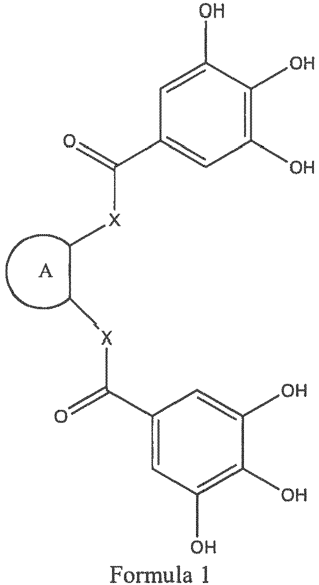 wherein X is O or NH, A is cyclopentyl, cyclohexyl, tetrahydrofuran, or tetrahydropyran, optionally substituted with one or more hydroxy, hydroxymethyl, or moiety of formula -X-(CO)-(3,4,5-trihydroxyphenyl); in free or in orally or topically acceptable salt form;
for example,
1.1. Composition 1 wherein X is O;
1.2. Composition 1 wherein X is NH;
1.3. Any of the foregoing compositions wherein the two moieties of formula -X-(CO)-(3,4,5-trihydroxyphenyl) on adjacent carbons in the gallate or gallamide compound are the *cis*-configuration relative to one another;
1.4. Any of the foregoing compositions wherein the two moieties of formula -X-(CO)-(3,4,5-trihydroxyphenyl) on adjacent carbons in the gallate or gallamide compound are in the *trans*-configuration relative to one another;
1.5. Any of the foregoing compositions wherein the gallate or gallamide compound is of Formula 1 and A is cyclohexyl or cyclopentyl;
1.6. Any of the foregoing compositions wherein the gallate or gallamide compound is selected from compounds of formulae 1a, formula 1b and mixtures thereof: 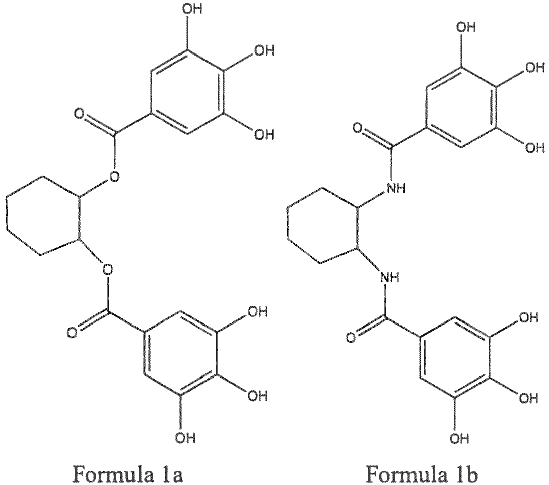
1.7. Any of the foregoing compositions wherein the compound of formula 1 is selected from *trans*-1,2 cyclohexanediol digallate (THDG), *cis*-1,2-cyclohexanediol digallate (CHDG), *cis*-1,2-cyclopentanediol digallate (CPDG), trans-1,2-cyclohexane digallamide (THDGA), and *cis*-1,2-cyclohexane digallamide (CHDGA): 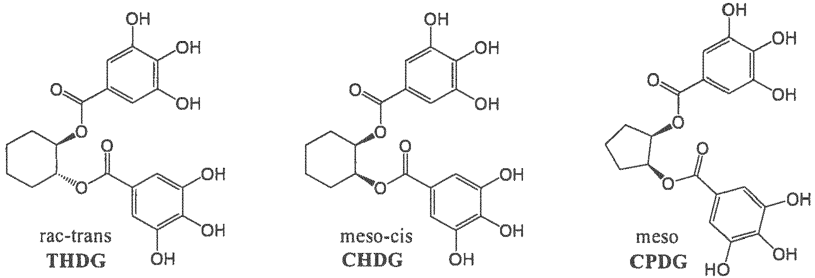 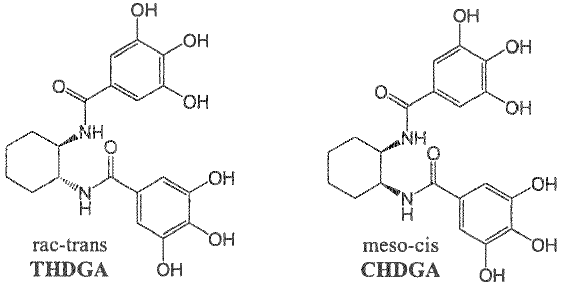 and mixtures thereof.
1.8. Any of the foregoing compositions further comprising a surfactant, an antioxidizing agent, and/or a buffer;
1.9. Any of the foregoing compositions in the form of an antimicrobial soap, ointment, cleanser or cream for topical application to the skin.
2. For example, the invention provides any of the foregoing Compositions which is an oral care composition (Composition 2), e.g.,
2.1. Composition 2 further comprising an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and combinations thereof;
2.2. Any of the foregoing compositions 2 et seq. comprising 1-arginine in free or orally acceptable salt form;
2.3. Any of the foregoing compositions 2 et seq. comprising buffering agents, e.g., sodium phosphate buffer (e.g., sodium phosphate monobasic and disodium phosphate)
2.4. Any of the foregoing compositions 2 et seq. comprising a humectant, e.g., selected from glycerin, sorbitol, propylene glycol, polyethylene glycol, xylitol, and mixtures thereof;
2.5. Any of the foregoing compositions 2 et seq. further comprising an abrasive or particulate;
2.6. The immediately preceding composition wherein the adhesive or particulate is selected from sodium bicarbonate, calcium phosphate (e.g., dicalcium phosphate dihydrate), calcium sulfate, precipitated calcium carbonate, silica (e.g., hydrated silica), iron oxide, aluminum oxide, perlite, plastic particles, e.g., polyethylene, and combinations thereof;
2.7. Any of the foregoing compositions 2 et seq. comprising an abrasive in an amount of about 15 wt. % to about 70 wt. % of the total composition weight;
2.8. Any of the foregoing compositions 2 et seq. comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight;
2.9. Any of the foregoing compositions 2 et seq. further comprising at least one polymer, e.g., selected from polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum), and combinations thereof;
2.10. Any of the foregoing compositions 2 et seq. comprising gum strips or fragments;
2.11. Any of the foregoing compositions 2 et seq. further comprising flavoring, fragrance and/or coloring;
2.12. Any of the foregoing compositions 2 et seq. further comprising water;
2.13. Any of the foregoing compositions 2 et seq. comprising one or more antibacterial agents in addition to the compound of formula 1, for example comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan or cetylpyridinium chloride;
2.14. Any of the foregoing compositions 2 et seq. further comprising an anti-inflammatory compound, e.g., an inhibitor of at least one of host pro-inflammatory factors selected from matrix metalloproteinases (MMP's), cyclooxygenases (COX), PGE2, interleukin 1 (IL-1), IL-1β converting enzyme (ICE), transforming growth factor β1 (TGF-β1), inducible nitric oxide synthase (iNOS), hyaluronidase, cathepsins, nuclear factor kappa B (NF-κB), and IL-1 Receptor Associated Kinase (IRAK), e,g, selected from aspirin, ketorolac, flurbiprofen, ibuprofen, naproxen, indomethacin, aspirin, ketoprofen, piroxicam, meclofenamic acid, nordihydoguaiaretic acid, and mixtures thereof;
2.15. Any of the foregoing compositions 2 et seq. further comprising an antioxidant, e.g., selected from the group consisting of Co-enzyme Q10, PQQ, Vitamin C, Vitamin E, Vitamin A, anethole-dithiothione, and mixtures thereof;
2.16. Any of the foregoing compositions 2 et seq. further comprising a whitening agent, e.g., a selected from the group consisting of peroxides, metal chlorites, perborates, percarbonates, peroxyacids, hypochlorites, and combinations thereof;
2.17. Any of the foregoing compositions 2 et seq. further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate);
2.18. Any of the foregoing compositions 2 et seq. further comprising an agent that interferes with or prevents bacterial attachment, e.g., solbrol or chitosan;
2.19. 1.30. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate;
2.20. Any of the foregoing compositions 2 et seq. further comprising a soluble calcium salt, e.g., selected from calcium sulfate, calcium chloride, calcium nitrate, calcium acetate, calcium lactate, and combinations thereof;
2.21. Any of the foregoing compositions 2 et seq. further comprising a physiologically acceptable potassium salt, e.g., potassium nitrate or potassium chloride, in an amount effective to reduce dentinal sensitivity;
2.22. Any of the foregoing compositions 2 et seq. further comprising a breath freshener, fragrance or flavoring;
2.23. Any of the foregoing compositions 2 et seq. effective upon application to the oral cavity, e.g., with brushing, to (i) inhibit microbial biofilm formation in the oral cavity, (ii) to reduce plaque accumulation, (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues;
2.24. A composition obtained or obtainable by combining the ingredients as set forth in any of the preceding compositions;
2.25. Any of the foregoing compositions 2 et seq. in a form selected from mouthrinse, toothpaste, tooth gel, tooth powder, non-abrasive gel, mousse, foam, mouth spray, lozenge, oral tablet, dental implement, and pet oral care product;
2.26. Any of the foregoing compositions 2 et seq. further comprising effective amounts of additional agents selected from fluoride, 1-arginine in free or orally acceptable salt form, antibacterial agents in addition to the compound of formula 1, anti-inflammatory compounds, and whitening agents;
2.27. Any of the foregoing compositions 2 et seq. wherein the composition is a toothpaste or mouthwash optionally further comprising one or more of one or more of water, abrasives, surfactants, foaming agents, vitamins, polymers, enzymes, humectants, thickeners, antimicrobial agents, preservatives, flavorings, colorings and/or combinations thereof;
2.28. Any of the foregoing compositions 2 et seq. wherein the composition is toothpaste;
2.29. Any of the foregoing compositions 2 -2.28 wherein the composition is a mouthwash.

In another embodiment, the invention provides methods to (i) inhibit microbial biofilm formation in the oral cavity, (ii) to reduce plaque accumulation, (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues; comprising applying to the oral cavity an effective amount of a composition comprising a compound of Formula 1, as hereinbefore described, e.g., an oral care compositions, for example any of Composition 2, et seq.

The invention further provides a method to reduce, inhibit, or treat topical or superficial microbial infections, for example to treat, reduce or inhibit topical skin infections, e.g., acne, superficial skin infections, minor cuts, pathogen colonization, and inflammatory skin conditions, comprising applying a composition comprising from 0.001 to 5% of a compound of Formula 1, e.g., Composition 1 et seq. to the affected skin or nails of a subject in need thereof.

The invention further provides the use of a compound of Formula 1 in the manufacture of a formulation for topical application, e.g., a Composition 1 et seq or 2 et seq, e.g., for use in any of the methods as described in the preceding two paragraphs, as well as methods of manufacturing an oral care composition, e.g., Composition 1, et seq., comprising combining a compound of formula 1 with an orally or topically acceptable carrier or vehicle.

*Orally or topically acceptable:* The compositions of the invention are intended for topical use on the skin or in the mouth, thus excipients for use in the present invention should be orally or topically acceptable, that is, safe for topical use in the mouth or on the skin, in the amounts and concentrations provided. Orally and topically acceptable formulation excipients are known in the art and are further exemplified herein. When compounds of the invention are capable of forming salts, e.g., acid or base addition salts, the salts for use in the present invention should be orally or topically acceptable, that is, safe for topical use in the mouth or skin, in the amounts and concentrations provided. Suitable salts include salts known in the art to be pharmaceutically acceptable salts, which are generally considered to be orally and topically acceptable for this purpose in the amounts and concentrations provided.

*Active Agents*: The concentration of the active ingredients used herein is from 0.001 to 5%. While the compounds of formula 1 do have intrinsic antibacterial activity, without being bound by theory, we believe that most of the antibacterial effect of the compounds of formula 1 when administered is due to their induction of the release of the antibacterial peptide LL-37 from epithelial cells. Thus, an antibacterial concentration of the compound of formula 1 is includes a concentration effective to induce release of the antibacterial peptide LL-37 from human epithelial cells. It is moreover understood that a toothpaste or soap for example will typically be diluted with water upon use, while a mouth rinse or topical pharmaceutical preparation typically will not be. The concentrations of a compound of formula 1 in a composition for use in the instant invention are thus from 0.001 to 5%, e.g., about 0.01-0.1% for a mouth rinse and about 0.1 to 1 % for a toothpaste.

Other actives, when present in compositions of the invention, are provided in effective amounts. Arginine, where present, may be present at levels from, e.g., about 0.1 to about 20 wt %(expressed as weight of free base), e.g., about 0.1 to about 3 wt % for a mouthrinse, about 1 to about 10 wt % for a consumer toothpaste or about 7 to about 20 wt % for a professional or prescription treatment product. Fluoride where present may be present at levels of, e.g., about 25 to about 25,000 ppm, for example about 25 to about 250 ppm for a mouthrinse, about 750 to about 2,000 ppm for a consumer toothpaste, or about 2,000 to about 25,000 ppm for a professional or prescription treatment product. Levels of antibacterial agents in addition to the compound of formula 1 will vary similarly, with levels used in toothpaste being e.g., about 5 to about 15 times greater than used in mouthrinse. For example, a triclosan mouthrinse may contain, e.g., about 0.03 wt % triclosan while a triclosan toothpaste may contain about 0.3 wt % triclosan.

*Fluoride Ion Source*: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A mouthwash, for example, would typically have about 100 to about 250 ppm fluoride. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt.

*Abrasives*: The compositions of the invention, e.g. Composition 2 et seq. may comprise a calcium phosphate abrasive, e.g., tricalcium phosphate (Ca₃(PO₄)₂), hydroxyapatite (Ca₁₀(PO₄)₆(OH₂), or dicalcium phosphate dihydrate (CaHPO₄ • 2H₂O, also sometimes referred to herein as DiCal) or calcium pyrophosphate. The compositions may include one or more additional abrasives, for example silica abrasives such as precipitated silicas having a mean particle size of up to about 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof. The silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention. The abrasive is present in the oral care composition of the present invention at a concentration of about 10 to about 60% by weight, in other embodiment about 20 to about 45% by weight, and in another embodiment about 30 to about 50% by weight.

The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants*: The compositions useful in the invention may contain anionic surfactants, for example
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂))₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01 % by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

In another embodiment, cationic surfactants useful in the present invention can be broadly defined as derivatives of aliphatic quaternary ammonium compounds having one long alkyl chain containing about 8 to about 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains about 8 to about 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Flavoring Agents*: The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight and about 0.5 to about 1.5% by weight. The dosage of flavoring agent in the individual oral care composition dosage (i.e., a single dose) is about 0.001 to 0.05% by weight and in another embodiment about 0.005 to about 0.015 % by weight.

*Polymers*: The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Efficacy of compounds of formula 1 and other noncationic antibacterial agents, e.g., triclosan, in a dentifrice may be enhanced by including from about 0.05 to about 5% of an agent which enhances the delivery and retention of the agents to, and retention thereof on oral surfaces. Such agents useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 30,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al. In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

*Enzymes:* The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191. An enzyme of a mixture of several compatible enzymes in the current invention constitutes about 0.002% to about 2.0% in one embodiment or about 0.05% to about 1.5% in another embodiment or in yet another embodiment about 0.1% to about 0.5%.

*Water:* Water may also be present in the oral compositions of the invention. Water, employed in the preparation of commercial oral compositions should be deionized and free of organic impurities. Water commonly makes up the balance of the compositions and includes about 10% to about 90%, about 20% to about 60% or about 10% to about 30% by weight of the oral compositions. This amount of water includes the free water which is added plus that amount which is introduced with other materials such as with sorbitol or any components of the invention.

*Humectants:* Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70% in one embodiment or about 30% to about 65% in another embodiment by weight of the dentifrice composition. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

*Other optional ingredients:* In addition to the above-described components, the embodiments of this invention can contain a variety of optional ingredients some of which are described below. Optional ingredients include, for example, but are not limited to sudsing agents, flavorings or fragrances, additional antiplaque or anti-biofilm agents, and coloring agents. These and other optional components are, for example, further described in U.S. Pat. No. 5,004,597, to Majeti; U.S. Pat. No. 3,959,458 to Agricola et al. and U.S. Pat. No. 3,937,807, to Haefele.

Synthesis of compounds of formula 1 is described in, e.g., Kazi, et al., Anticancer Res. (2004) 24: 943-954,WO 2010/043631, and US 2010/0137194. For example, the compounds can be made by a conventional Schotten-Baumann reaction, reacting the desired polyhydroxy or polyamino scaffold, e.g., 1,2-dihydroxy-cyclohexane, 1,2-dihydroxy-cyclopentane, 1,2-diamino-cyclohexane, 1,2-diamino-cyclopentane, furanose or pyranose, with gallic acid in O-protected acid chloride form, recovering the O-protected gallic acid ester or amide compound formed thereby, and deprotecting to obtain the compounds of Formula 1.

As used throughout, ranges are used as shorthand for describing each and every value that is within the range. Any value within the range can be selected as the terminus of the range. In the event of a conflict in a definition in the present disclosure and that of a cited reference, the present disclosure controls. Unless otherwise specified, all percentages and amounts expressed herein and elsewhere in the specification should be understood to refer to percentages by weight.

### EXAMPLES

### Example 1

Based on molecular modeling and preliminary screens, the following gallate or gallamide compounds (esters and amides) are moved forward for further evaluation:

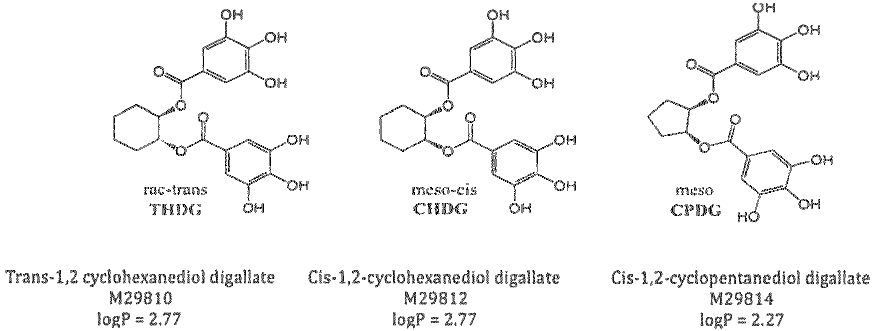

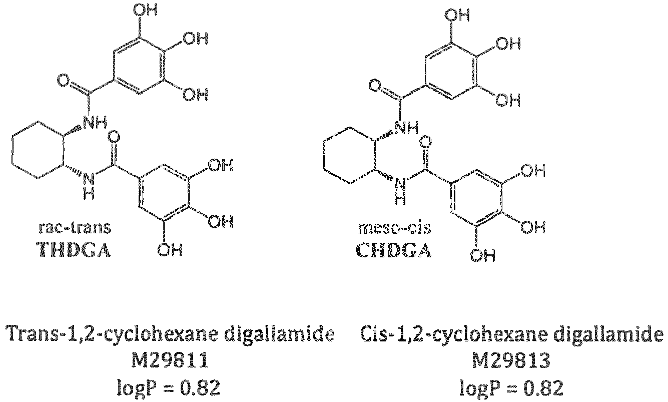

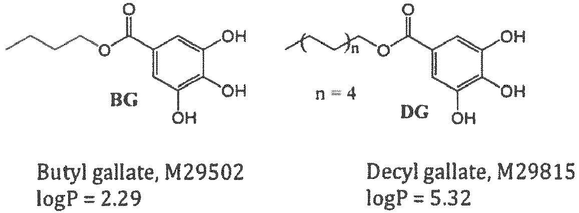

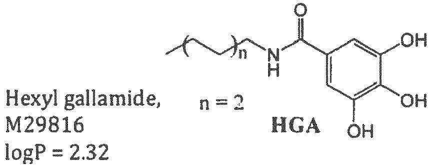

*Antioxidant assay:* The compounds are tested using a commercially available antioxidant assay kit (Sigma, catalog no. CS0790). The principle of the antioxidant assay is formation of a ferryl myoglobin radical from metmyoglobin and hydrogen peroxide, which oxidizes the ABTS (2,2' -azino-bis (3-ethylbenzthiazoline-6-sulfonic acid) to produce a radical cation ABTS⁺ a soluble chromogen that is green in color and can be determined spectrophotometrically at 405 nm.

HX-FE^{III} + H₂O₂ → X - (Fe^{IV}=O) + H₂O

ABTS + X - (Fe^{IV} =O) → ABTS⁺ + HX-Fe^{III}

In this equation, HX-Fe^{III} is metmyoglobin and X - (Fe^{IV}=O) is ferryl myoglobin. Metmyoglobin is oxidized by hydrogen peroxide to ferryl myoglobin, which in turn oxidizes the dye ABTS into the radical cation ABTS⁺. Antioxidants suppress the production of the radical cation in a concentration dependent manner and the color intensity decreases proportionally, meaning that less green in this assay in the presence of a test compound corresponds with better antioxidant activity by the test compound. Trolox® (6-hydroxy-2,5,7,8-tetramethylchroman-2-carboxylic acid, a water-soluble derivative of vitamin E known to be a potent antioxidant) serves as a control or standard antioxidant. Antioxidant activity is expressed in terms of equivalence to mM Trolox®. All of the compounds tested except DG show activity equal to or exceeding EGCG.

**Table 1**

| **Name of Analog** | **Concentration (mM)** | **Antioxidant Equivalence in mM Trolox** |
|---|---|---|
| CHDG | 2.38 | 7.3 |
| THDG | 2.38 | 7.6 |
| CPDG | 2.46 | 6.9 |
| CHDGA | 2.39 | 6.7 |
| THDGA | 2.39 | 6.7 |
| BG | 4.42 | 8.4 |
| DG | 0.64 | 0.8 |
| HGA | 3.96 | 6.5 |
| EGCG | 1.96 | 5.6 |

*Formulation suitability:* The compounds are assessed for stability in storage. None of the test compounds exhibit the browning seen with EGCG. The compounds are white to yellow powders. Fresh solutions in DMSO/water are colorless. No discoloration is seen in 1M at room temperature, apart from a slight yellowing for HGA. The test compounds are generally lipophilic and less soluble in water than EGCG.

*Antibacterial assay:* A growth inhibition (GI) assay is performed to determine the ability of the EGCG analogs in inhibiting bacterial growth. An overnight culture of *Actinomyces viscosus* (ATCC # 43146) is diluted to an OD610 ∼ 0.2 in sterile trypticase soy broth (TSB) Nine mls of diluted *Actinomyces viscosus* is added to triplicate 15 mls sterile culture tubes for each sample or control tested. One ml of each analog was added to the corresponding culture tube and briefly vortexed. Cultures are incubated at 37°C in a shaking water bath. Supernatants are removed at 2, 4, 6, and 24h and the OD610 read. Results from triplicate samples were averaged and growth is plotted as a function of time. Each of the above compounds has modest antibacterial activity in bacterial culture at 100 ppm, the same as EGCG, with the exception of DG, which has activity at 20 ppm.

*Induction of Anti-Microbial Peptides (AMP)*: Human gingival epithelial cells are grown and treated with test compounds at 0.1% and 0.05% solution (DG is tested at lower concentrations because of its poor solubility) at 4 and 16 hours, supernatants are collected and tested for AMP levels by ELISA for LL-37 and β-defensin.

**Table 2**

| **Test compound** | **LL-37 (ng/ml) at 0.1% (0.025% for DG) after 4 hrs** | **LL-37 (ng/ml) at 0.05% (0.01% DG) after 16 hrs** | **β-defensin (ng/ml) at 0.1% (0.025% for DG) after 4 hrs** |
|---|---|---|---|
| CHDG | 5200 | 3700 | 150 |
| THDG | 4100 | 5900 | 280 |
| CPDG | 120 | 160 | 0.5 |
| CHDGA | 100 | 120 | 0.7 |
| THDGA | 100 | 130 | 1.5 |
| BG | 100 | 50 | 0 |
| DG | 110 | 10 | 0 |
| HGA | 180 | 0 | 0.4 |
| EGCG (source TeaVigo) | 160 | 20 | 4.3 |
| EGCG (source Kenko) | 160 | 15 | -- |

At 0.05% concentration, test compounds having a cyclohexyl or cyclopropyl scaffold are more potent than EGCG in inducing expression of LL-37, and have a sustained effect on LL-37 induction. Induction of defensin expression is weaker than induction of LL-37 expression for all compounds tested. Accordingly, these compounds would have significant antibacterial activity in the mouth, in excess of their intrinsic antibacterial activity, by virtue of their ability to induce epithelial cells to release the antibacterial peptide LL-37.

### Example 2

### Dical base - CDC formula

Selected compounds are added to conventional toothpastes to determine stability, as follows:
0.2% THDG in silica base, comprising 1.8% SLS with fluoride (HSLS)
0.2% THDG in Dical base (CDC)
0.2% CHDG in silica base, comprising 1.8% SLS with fluoride
0.2% CHDG in Dical base

Formulations are made by post-addition and mixing in a Speed Mixer. Samples are aged at ambient conditions, no pH control, no stabilizers added. Recovery of test compound is measured after one and two weeks:

| | 0.2% in CDC | | 0.2% in HSLS | |
|---|---|---|---|---|
| Recovery of | 1 week RT | 2 weeks RT | 1 week RT | 2 weeks RT |
| THDG | 90% | 84% | 79% | n/a |
| CHDG | n/a | 87% | 91% | n/a |

The stability of THDG and CHDG is assessed as follows: (i) Stability of neat materials: white to off white powders, short term shelf stability acceptable; (ii) stability of CHDG/THDG solutions in DMSO/water: pH 6.1 - 6.5, no discoloration after 4M at RT vs. Teavigo EGCG under the same conditions - discolored in < 1 week; (iii) stability in saliva (24 h at 37°C): no discoloration to deep green discoloration - subject specific, depending on saliva pH (6.5 - 8); (iv) stability in dentrifice prototypes: for dical base, slight discoloration can be visible for THDG and CHDG prototypes after 2 weeks, while for Teavigo (EGCG) prototype, this happens in only 2 days; for silica base, moderate discoloration can be visible for THDG/CHDG prototypes after 1 week, while for Teavigo prototype, this happens in only 2 days. Thus, while some optimization is required for consumer acceptable product, the claimed compounds nevertheless show significant stability advantages over EGCG.

Using a commercial Vitro-Skin® assay to measure potential uptake in skin, we see uptake from all the formulations, but have better uptake in the Dical formulation than in the silica/SLS formulation (units in micrograms/cm²):
0.2% THDG/CDC: 4.9
0.2% THDG/HSLS: 0.6
0.2% CHDG/CDC: 4.4
0.2% CHDG/CDC: 0.8
0.3% Triclosan (positive control): 7.7

Thus, even without formulation optimization, the compounds show reasonable stability and delivery in formulation, and are significantly better in terms of stability than EGCG.

## Claims

1. An oral care composition for topical use comprising a gallate or gallamide compound which is a polysubstituted cycloalkyl, wherein the substituents are selected from hydroxy, hydroxymethyl, fluoro, chloro, amino, nitro, or a moiety of formula -X-(CO)-(3,4,5-trihydroxyphenyl), wherein X is selected from O and NH, provided that the substituents comprise at least two moieties of formula-X-(CO)-(3,4,5-trihydroxyphenyl) attached to adjacent carbons; in free or in orally or topically acceptable salt form, wherein the concentration of the gallate or gallamide compound is from 0.001 to 5%.

2. The composition of claim I wherein the gallate or gallamide compound is a compound of Formula 1: 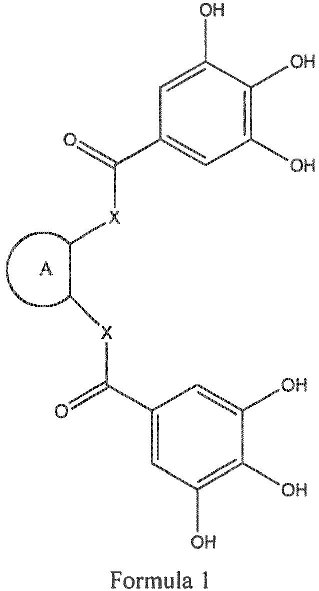 wherein X is O or NH, A is cyclopentyl or cyclohexyl, optionally substituted with one or more substituents selected from one or more of hydroxy, hydroxymethyl, and moiety of formula -X-(CO)-(3,4,5-trihydroxyphenyl); in free or in orally or topically acceptable salt form.

3. The composition according to claim 1 wherein X is O, and/or wherein X is NH.

4. The composition of claims 1 to 3, wherein A is cyclohexyl or cyclopentyl.

5. The composition of claims 1 to 4, wherein the gallate or gallamide compound is selected from *trans*-1,2 cyclohexanediol digallate (THDG), *cis*-1,2-cyclohexanediol digallate (CHDG), *cis*-1,2-cyclopentanediol digallate (CPDG), *trans*-1,2-cyclohexane digallamide (THDGA), and *cis*-1,2-cyclohexane digallamide (CHDGA) and mixtures thereof, preferably wherein the gallate or gallamide compound is selected from *trans-1,2* cyclohexanediol digallate (THDG), *cis*-1,2-cyclohexanediol digallate (CHDG) and mixtures thereof.

6. The composition of any foregoing claim further comprising a surfactant, an antioxidizing agent, and/or a buffer.

7. The composition of any preceding claim, wherein the composition is a toothpaste or mouthwash further comprising one or more of water, an abrasive, a surfactant, a foaming agent, a vitamin, a polymer, an enzyme, a humectant, a thickener, an antimicrobial agent, a preservative, a flavoring, a coloring and/or combinations thereof, preferably wherein the composition comprises an abrasive, and said abrasive is present in an amount of about 15 wt. % to about 70 wt. % of the total composition weight.

8. The composition of any preceding claim, which is an oral care composition further comprising an effective amount of an additional agent selected from a fluoride ion source, arginine in free or orally acceptable salt form, an antibacterial agent in addition to a compound of formula 1, an anti-inflammatory agent, a whitening agent, and a combination of two or more thereof.

9. The composition according to any one of claims 1-8 for use in (i) inhibiting microbial biofilm formation in the oral cavity, (ii) reducing plaque accumulation, (iii) reducing or inhibiting gingivitis, (iv) reducing or inhibiting formation of dental caries, (v), reducing, repairing or inhibiting pre-carious lesions of the enamel, (vi) cleaning the teeth and oral cavity, and/or (vii) promoting systemic health.

10. The composition for use according to claim 9 wherein the formulation for topical application is an oral care formulation selected from a toothpaste and a mouthrinse.

11. A composition for topical use comprising a gallate compound which is a polysubstituted cycloalkyl wherein the substituents are selected from hydroxy, hydroxymethyl, fluoro, chloro, amino, nitro, or a moiety of formula -X-(CO)-(3,4,5-trihydroxyphenyl), wherein X is selected from O, provided that the substituents comprise at least two moieties of formula-X-(CO)-(3,4,5-trihydroxyphenyl) attached to adjacent carbons; in free or in topically acceptable salt form, wherein the concentration of the gallate or gallamide compound is from 0.001 to 5%.

12. The composition of claim 11 wherein the gallate compound is a compound of Formula 1: 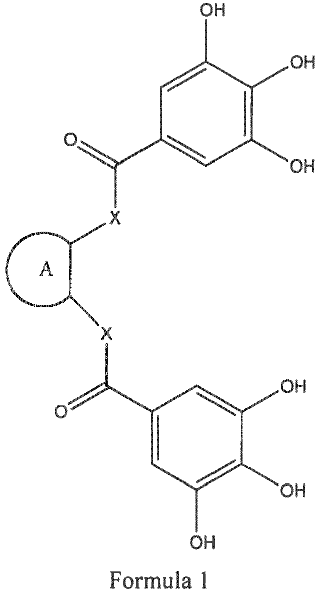 wherein X is O, A is cyclopentyl or cyclohexyl, optionally substituted with one or more substituents selected from one or more of hydroxy, hydroxymethyl, and moiety of formula -X-(CO)-(3,4,5-trihydroxyphenyl); in free or topically acceptable salt form.

13. The composition of claims 11 or 12, wherein A is cyclohexyl or cyclopentyl, and/or wherein the gallate compound is selected from *trans*-1,2 cyclohexanediol digallate (THDG), *cis*-1,2-cyclohexanediol digallate (CHDG), *cis*-1,2-cyclopentanediol digallate (CPDG), *trans-1,2-cyclohexane* digallamide (THDGA), and *cis*-1,2-cyclohexane digallamide (CHDGA) and mixtures thereof, preferably wherein the gallate compound is selected from *trans*-1,2 cyclohexanediol digallate (THDG), *cis*-1,2-cyclohexanediol digallate (CHDG) and mixtures thereof.

14. The composition of any of claims 11 to 13 further comprising a surfactant, an antioxidizing agent, and/or a buffer, and/or wherein the compositions is in the form of an antimicrobial soap, ointment, cleanser or cream for topical application to the skin.

15. The composition according to any one of claims 11-14 for use in reducing, inhibiting, or treating topical or superficial microbial infections, optionally acne, superficial skin infections, minor cuts, pathogen colonization, and inflammatory skin conditions.

## Patentansprüche

1. Eine Mundpflegezusammensetzung zur topischen Anwendung, die eine Gallat- oder Gallamidverbindung beinhaltet, die ein polysubstituiertes Cycloalkyl ist, wobei die Substituenten aus Hydroxy, Hydroxymethyl, Fluoro, Chloro, Amino, Nitro oder einem Anteil der Formel -X-(CO)-(3,4,5-trihydroxyphenyl) ausgewählt sind, wobei X aus O und NH ausgewählt ist, vorausgesetzt, die Substituenten beinhalten mindestens zwei Anteile der Formel -X-(CO)-(3,4,5-trihydroxyphenyl), angehängt an benachbarte Kohlenstoffe; in freier Form oder in Form eines oral oder topisch akzeptablen Salzes, wobei die Konzentration der Gallat- oder Gallamidverbindung von 0,001 bis 5% beträgt.

2. Die Zusammensetzung gemäß Anspruch 1, wobei die Gallat- oder Gallamidverbindung eine Verbindung der Formel 1 ist: 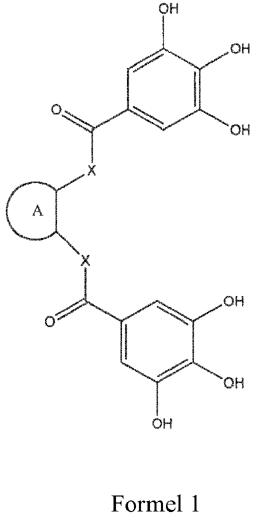 wobei X O oder NH ist, A Cyclopentyl oder Cyclohexyl ist, optional substituiert mit einem oder mehreren Substituenten, ausgewählt aus Hydroxy, Hydroxymethyl und einem Anteil der Formel -X-(CO)-(3,4,5-trihydroxyphenyl); in freier Form oder in Form eines oral oder topisch akzeptablen Salzes.

3. Die Zusammensetzung gemäß Anspruch 1, wobei X O ist und/oder wobei X NH ist.

4. Die Zusammensetzung gemäß Ansprüchen 1 bis 3, wobei A Cyclohexyl oder Cyclopentyl ist.

5. Die Zusammensetzung gemäß Anspruch 1 bis 4, wobei die Gallat- oder Gallamidverbindung aus *trans*-1,2-Cyclohexandioldigallat (THDG), *cis*-1,2-Cyclohexandioldigallat (CHDG), *cis*-1,2-Cyclopentandioldigallat (CPDG), *trans*-1,2-Cyclohexandigallamid (THDGA) und *cis*-1,2-Cyclohexandigallamid (CHDGA) und Mischungen davon ausgewählt ist, wobei die Gallat- oder Gallamidverbindung vorzugsweise aus *trans*-1,2-Cyclohexandioldigallat (THDG), *cis*-1,2-Cyclohexandioldigallat (CHDG) und Mischungen davon ausgewählt ist.

6. Die Zusammensetzung gemäß einem der vorhergehenden Ansprüche, die ferner ein Tensid, ein Antioxidationsmittel und/oder einen Puffer beinhaltet.

7. Die Zusammensetzung gemäß einem der vorhergehenden Ansprüche, wobei die Zusammensetzung eine Zahnpasta oder ein Mundwasser ist, die ferner eines oder mehrere von Wasser, Scheuermitteln, Tensiden, Schäumungsmitteln, Vitaminen, Polymeren, Enzymen, Feuchthaltemitteln, Verdickungsmitteln, antimikrobiellen Mitteln, Konservierungsmitteln, Geschmacksstoffen, Farbstoffen und/oder Kombinationen davon beinhaltet, wobei die Zusammensetzung vorzugsweise ein Scheuermittel beinhaltet und das Scheuermittel in einer Menge von etwa 15 Gew.-% bis etwa 70 Gew.-% des Gesamtgewichts der Zusammensetzung vorliegt.

8. Die Zusammensetzung gemäß einem der vorhergehenden Ansprüche, die eine Mundpflegezusammensetzung ist, die ferner eine wirksame Menge eines zusätzlichen Mittels beinhaltet, das aus einer Fluoridionenquelle, Arginin in freier Form oder Form eines oral akzeptablen Salzes, einem antibakteriellen Mittel zusätzlich zu einer Verbindung der Formel 1, einem entzündungshemmenden Mittel, einem Weißmacher und einer Kombination von zwei oder mehreren davon ausgewählt ist.

9. Die Zusammensetzung gemäß einem der Ansprüche 1-8 zur Verwendung beim (i) Hemmen der Bildung von mikrobiellem Biofilm in der Mundhöhle, (ii) Verringern der Ansammlung von Plaque, (iii) Verringern oder Hemmen von Gingivitis, (iv) Verringern oder Hemmen der Bildung von Zahnkaries, (v) Verringern, Reparieren oder Hemmen präkariöser Läsionen des Zahnschmelzes, (vi) Reinigen der Zähne und der Mundhöhle und/oder (vii) Fördern der systemischen Gesundheit.

10. Die Zusammensetzung gemäß Anspruch 9, wobei die Formulierung zur topischen Applikation eine Mundpflegeformulierung ist, die aus einer Zahnpasta und einer Mundspülung ausgewählt ist.

11. Eine Zusammensetzung zur topischen Anwendung, die eine Gallatverbindung beinhaltet, die ein polysubstituiertes Cycloalkyl ist, wobei die Substituenten aus Hydroxy, Hydroxymethyl, Fluoro, Chloro, Amino, Nitro oder einem Anteil der Formel-X-(CO)-(3,4,5-trihydroxyphenyl) ausgewählt sind, wobei X aus O ausgewählt ist, vorausgesetzt, die Substituenten beinhalten mindestens zwei Anteile der Formel -X-(CO)-(3,4,5-trihydroxyphenyl), angehängt an benachbarte Kohlenstoffe; in freier Form oder in Form eines topisch akzeptablen Salzes, wobei die Konzentration der Gallat- oder Gallamidverbindung von 0,001 bis 5% beträgt.

12. Die Zusammensetzung gemäß Anspruch 11, wobei die Gallatverbindung eine Verbindung der Formel 1 ist: 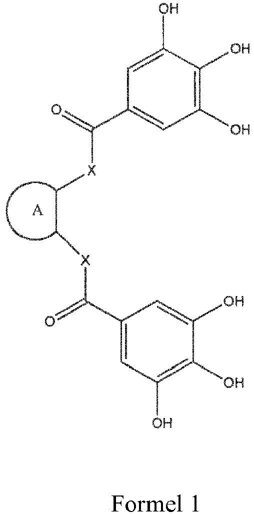 wobei X O ist, A Cyclopentyl oder Cyclohexyl ist, optional substituiert mit einem oder mehreren Substituenten, ausgewählt aus einem oder mehreren von Hydroxy, Hydroxymethyl und einem Anteil der Formel -X-(CO)-(3,4,5-trihydroxyphenyl); in freier Form oder in Form eines topisch akzeptablen Salzes.

13. Die Zusammensetzung gemäß Ansprüchen 11 oder 12, wobei A Cyclohexyl oder Cyclopentyl ist und/oder wobei die Gallatverbindung aus *trans*-1,2-Cyclohexandioldigallat (THDG), *cis*-1,2-Cyclohexandioldigallat (CHDG), *cis*-1,2-Cyclopentandioldigallat (CPDG), *trans*-1,2-Cyclohexandigallamid (THDGA) und *cis*-1,2-Cyclohexandigallamid (CHDGA) und Mischungen davon ausgewählt ist, wobei die Gallatverbindung vorzugsweise aus *trans*-1,2-Cyclohexandioldigallat (THDG), *cis*-1,2-Cyclohexandioldigallat (CHDG) und Mischungen davon ausgewählt ist

14. Die Zusammensetzung gemäß einem der Ansprüche 11 bis 13, die ferner ein Tensid, ein Antioxidationsmittel und/oder einen Puffer beinhaltet und/oder wobei die Zusammensetzung in Form einer antimikrobiellen Seife, Salbe, eines Reinigers oder einer Creme zur topischen Applikation auf der Haut ist.

15. Die Zusammensetzung gemäß einem der Ansprüche 11-14 zur Verwendung beim Verringern, Hemmen oder Behandeln von topischen oder oberflächlichen mikrobiellen Infektionen, optional von Akne, oberflächlichen Hautinfektionen, kleinen Schnittwunden, von Besiedlung durch Erreger und von entzündlichen Hauterkrankungen.

## Revendications

1. Composition pour soin buccal destinée à un usage topique comprenant un composé de gallate ou de gallamide qui est un cycloalkyle polysubstitué, dans laquelle les substituants sont sélectionnés parmi l'hydroxy, l'hydroxy méthyle, le fluoro, le chloro, l'amino, le nitro ou une fraction de formule -X-(CO)-(3,4,5-trihydroxyphényle), dans laquelle X est sélectionné parmi O et NH, à condition que les substituants comprennent au moins deux fractions de formule -X-(CO)-(3,4,5-trihydroxyphényl) liées à des carbones adjacents ; sous forme libre ou sous forme de sel buccalement ou topiquement acceptable, dans laquelle la concentration en composé de gallate ou de gallamide est comprise dans l'intervalle allant de 0,001 à 5 %.

2. Composition selon la revendication 1 dans laquelle le composé de gallate ou de gallamide est un composé de formule 1 : 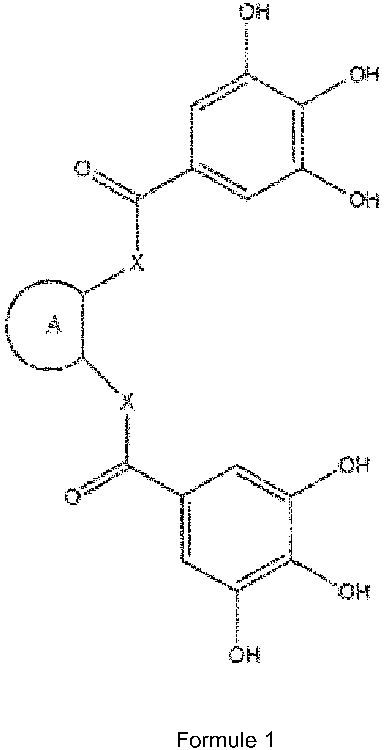 où X représente O ou NH, A représente le cyclopentyle ou le cyclohexyle, éventuellement substitué par un ou par plusieurs substituants sélectionnés parmi l'hydroxy, l'hydroxyméthyle, et une fraction de formule -X-(CO)-(3,4,5-trihydroxyphényle) ; sous forme libre ou sous forme buccalement ou topiquement acceptable.

3. Composition selon la revendication 1 dans laquelle X représente O, et/ou dans laquelle X représente NH.

4. Composition selon les revendications 1 à 3, dans laquelle A représente le cyclohexyle ou le cyclopentyle.

5. Composition selon les revendications 1 à 4, dans laquelle le composé de gallate ou de gallamide est sélectionné parmi le *trans*-1,2 cyclohexanédiol digallate (THDG), le *cis*-1,2-cyclohexanédiol digallate (CHDG), le *cis*-1,2-cyclopentanédiol digallate (CPDG), la *trans-1,2-cyclohexane* digallamide (THDGA), et la *cis*-1,2-cyclohexane digallamide (CHDGA) et des mélanges de ceux-ci, préférablement dans laquelle le composé de gallate ou de gallamide est sélectionné parmi le *trans*-1,2 cyclohexanédiol digallate (THDG), le *cis*-1,2-cyclohexanédiol digallate (CHDG) et des mélanges de ceux-ci.

6. Composition selon l'une quelconque des revendications précédentes comprenant en outre un agent tensio-actif, un agent antioxydant et/ou un tampon.

7. Composition selon l'une quelconque des revendications précédentes, ladite composition étant une pâte dentifrice ou un rince-bouche comprenant en outre un ou plusieurs éléments parmi l'eau, un abrasif, un agent tensio-actif, un agent moussant, une vitamine, un polymère, une enzyme, un humectant, un agent épaississant, un agent antimicrobien, un agent conservateur, un agent aromatisant, un agent colorant et/ou des combinaisons de ceux-ci, ladite composition comprenant de préférence un abrasif, et ledit abrasif étant présent en une quantité d'environ 15 % en poids à environ 70 % en poids du poids total de la composition.

8. Composition selon l'une quelconque des revendications précédentes, qui est une composition pour soin buccal comprenant en outre une quantité efficace d'un agent supplémentaire sélectionné parmi une source d'ions fluorure, une arginine sous forme libre ou sous forme de sel buccalement acceptable, un agent antibactérien en plus d'un composé de formule 1, un agent anti-inflammatoire, un agent de blanchiment et une combinaison de deux ou de plusieurs de ceux-ci.

9. Composition selon l'une quelconque des revendications 1 à 8 pour utilisation dans (i) l'inhibition de la formation de biofilm microbien dans la cavité buccale, (ii) la réduction de l'accumulation de la plaque dentaire, (iii) la réduction ou l'inhibition de la gingivite, (iv) la réduction ou l'inhibition de la formation de caries dentaires, (v) la réduction, la réparation ou l'inhibition de lésions pré-cariées de l'émail, (vi) le nettoyage des dents et de la cavité buccale, et/ou (vii) la promotion de la santé systémique.

10. Composition pour utilisation selon la revendication 9 dans laquelle la formulation pour application topique est une formulation pour soin buccal sélectionnée parmi une pâte dentifrice et un rince-bouche.

11. Composition pour utilisation topique comprenant un composé de gallate qui est un cycloalkyle polysubstitué dans laquelle les substituants sont sélectionnés parmi l'hydroxy, l'hydroxyméthyle, le fluoro, le chloro, l'amino, le nitro ou une fraction de formule -X-(CO)-(3,4,5-trihydrophényle), dans laquelle X est sélectionné parmi O, à condition que les substituants comprennent au moins deux fractions de formule -X-(CO)-(3,4,5-trihydrophényle) liées à des carbones adjacents ; sous forme libre ou sous forme de sel topiquement acceptable, la concentration du composé de gallate ou de gallamide étant comprise dans l'intervalle allant de 0,001 à 5%.

12. Composition selon la revendication 11 dans laquelle le composé de gallate est un composé de formule 1 : 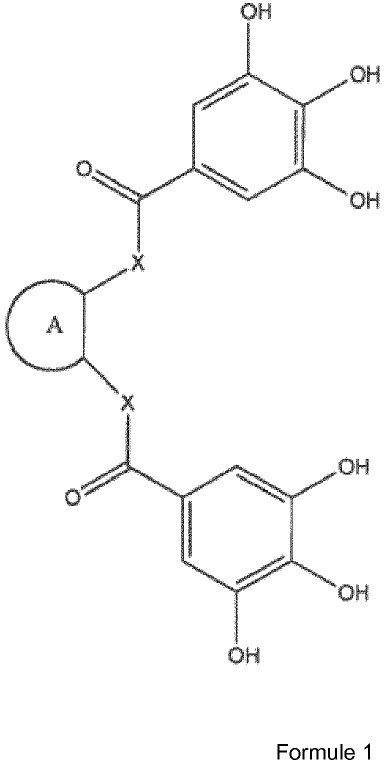 dans laquelle X représente O, A représente le cyclopentyle ou le cyclohexyle, éventuellement substitué par un ou plusieurs substituants sélectionnés parmi l'hydroxy, l'hydroxyméthyle et une fraction de formule X-(CO)-(3,4,5-trihydroxyphényl) ; sous forme libre ou sous forme de sel topiquement acceptable.

13. Composition selon les revendications 11 ou 12, dans laquelle A représente le cyclohexyle ou le cyclopentyle, et/ou dans laquelle le composé de gallate est sélectionné parmi le *trans*-1,2 cyclohexanédiol digallate (THDG), le *cis-*1,2-cyclohexanédiol digallate (CHDG), le *cis*-1,2-cyclopentanédiol digallate (CPDG), la *trans-1,2-cyclohexane* digallamide (THDGA), et la *cis*-1,2-cyclohexane digallamide (CHDGA) et des mélanges de ceux-ci, préférablement dans laquelle le composé de gallate est sélectionné parmi le *trans*-1,2 cyclohexanédiol digallate (THDG), le *cis*-1,2-cyclohexanédiol digallate (CHDG) et des mélanges de ceux-ci.

14. Composition selon l'une quelconque des revendications 11 à 13 comprenant en outre un agent tensio-actif, un agent antioxydant et/ou un tampon, et/ou ladite composition étant sous la forme d'un savon antimicrobien, d'un baume, d'un nettoyant ou d'une crème pour application topique sur la peau.

15. Composition selon l'une quelconque des revendications 11 à 14 pour utilisation dans la réduction, l'inhibition ou le traitement d'infections microbiennes topiques ou superficielles, éventuellement de l'acné, des infections cutanées superficielles, des coupures légères, de la colonisation par agents pathogènes et de conditions cutanées inflammatoires.
